# EUROPEAN PATENT APPLICATION

(11) **EP 1 435 359 A1**
(43) Date of publication of application: **07.07.2004**
(21) Application number: 03258239.7
(22) Date of filing: 30.12.2003
(51) Int. Cl.: C07H 17/08, A61K 31/70, A61P 31/04

(54) **A process for the purification of roxithromycin**

(30) Priority: 31.12.2002 IN MU11722002
(71) Applicant: Alembic Limited, Vadodara 390003, Gujarat (IN)
(72) Inventor: Mistry, Dhirenkumar N. Alembic Limited, Vadodara 390003, Gujarat (IN); Thorat, Mahadeo M. Alembic Limited, Vadodara 390003, Gujarat (IN); Soni, Kamlesh S. Alembic Limited, Vadodara 390003, Gujarat (IN); Mishra, Rakesh R. Alembic Limited, Vadodara 390003, Gujarat (IN); Kansal, Vinod K. Alembic Limited, Vadodara 390003, Gujarat (IN)
(74) Representative: Harrison Goddard Foote

(57) **Abstract**

A process for the purification of roxithromycin with high level of impurity -H comprising subjecting the impure roxithromycin to at least partial dissolution and/or suspension in solvent selected from ethanol, acetonitirle, aromatic solvents and mixture of ethanol and acetonitrile; followed by crystallizing to thereby obtain roxithromycin with desired level of impurity-H of less than 0.5%. The invention also relates to crystalline polymorphic forms B and C of roxithromycin obtained following the above process of purification and having impurity-H of less than 0.5%.

## Description

### FIELD OF INVENTION:

The present invention refers to a process for the purification of roxithromycin and in particular, purification of roxithromycin having high levels of impurity H and thereby provide pure roxithromycin of desired standards especially with impurity H within allowable limits as per the European Pharmacopoeia. The invention is further directed to novel polymorphs obtained following the above process of purification of roxithromycin.

### BACKGROUND AND PRIOR ART:

AT9400151 describes a process for the purification of roxithromycin by crystallisation with a halogenated solvent, namely chloroform. The usage of chloroform at an industrial level is not desirable as it is considered a hazardous solvent for the environment and, also not advisable to use it in the last step of the synthesis since it is considered as potentially carcinogenic.

US Patent 4349545 describes a process for the purification of roxithromycin by crystallisation with acetone and water, where the product is dissolved in warm acetone and the crystallisation caused by addition water. Mixture of acetone and water are not efficient in the reduction of late eluting impurity such as Impurity-H, which appears after the roxithromycin peak in the high-pressure liquid chromatography system of the European Pharmacopoeia. The reduction of this impurity is crucial since the European Pharmacopoeia establishes an individual limit of impurities at 0.5% and this impurity may have levels higher than 1% in crude roxithromycin. The process in the above-mentioned patent result in the preparation of a crystalline form of roxithromycin, which is herein, designated as Form A.

ES2026824 and ES2024371 describe processes for the purification of roxithromycin by column chromatography, which is expensive, impractical & tedious when, applied to an industrial scale.

EP 0985680 describes a process for the purification of roxithromycin by crystallisation with methanol and water. Although it is possible to remove other impurities such as impurity A by the use of methanol but higher level of impurity-H cannot be removed using Methanol alone or in combination with H₂O. Furthermore, IR of the roxithromycin prepared by the purification with methanol does not match with the EP reference standard in KBr pallet hence may also affect the bioequivalence. Moreover, methanol is toxic and has related problems.

The above clearly go to show the need to provide Roxithromycin with impurity H not exceeding 0.5% and the existing problems in obtaining Roxithromycin with such low levels of late eluting impurity 'H' following the conventional purification solvents involving methanol and acetone. Moreover, the conventional processes and the Roxithromycin forms obtained therefor were found to have problems of thermal stability and/or solubility, which again limited its widespread application and use.

The present invention provides a new method for purifying roxithromycin. More preferably, the invention aims to overcome or mitigate the drawbacks and shortcomings of the above discussed prior art in providing the desired roxithromycin form with low levels (e.g. of only up to 0.5%) of the late eluting impurity 'H'.

In one aspect the present invention is directed to a method of purification of roxithromycin having high impurity H, which method would provide roxithromycin with desired purity and especially a low level of impurity H as per the European Pharmacopoeia.

A yet further aspect of the present invention relates to the provision of novel polymorphs of roxithromycin, in particular high purity polymorphs which would meet the prescribed standards of the European pharmacopoeia reference standard with good thermal stability and/or solubility characteristics.

Further included is a method for conversion of the undesired polymorphs of roxithromycin to desired polymorph, in particular as provided by European pharmacopoeia reference standard.

### SUMMARY OF INVENTION:

Thus according to one aspect of the present invention there is provided a process for the purification of roxithromycin to reduce the level of impurity -H comprising dissolving the roxithromycin in a solvent described herein and then crystallizing the roxithromycin. Included is a process comprising:
(i) subjecting the roxithromycin to at least partial dissolution and/or suspension and optionally to total dissolution in a solvent selected from ethanol, acetonitrile, aromatic solvents and mixtures of ethanol and acetonitrile;
(ii) followed by crystallization to thereby obtain roxithromycin with a desired level of impurity-H, for example of less than 0.5%.

In accordance with a preferred aspect in said step of at least partial dissolution/suspension, it is possible to add a non-solvent (i.e. a liquid in which roxithromycin is substantially insoluble), such as water in case of ethanol, and/or the mix is concentrated to facilitate obtaining a purified product.

Importantly, the above process of purification of the invention is especially directed to limit the impurity-H to a level of less than 0.5% as required under the European Pharmacopoeia. While the prior art methods of purification and the solvents used such as methanol, chloroform, acetone provided for purification of roxithromycin, the same were found not suitable for the removal of high level of impurity H which, as per the European pharmacopoeia, is supposed to be limited to 0.5%. It is now found by way of the present invention that the selective solvent ethanol, acetonitrile, aromatic solvents and mixtures of ethanol and acetonitrile can provide for selective removal of impurity-H from roxithromycin.

In accordance with another aspect of the present invention there is provided a process for producing high purity polymorphs of roxithromycin comprising:
(i) subjecting the impure roxithromycin to at least partial dissolution and/or suspension in solvent selected from ethanol, acetonitrile, aromatic solvents and mixtures of ethanol and acetonitrile;
(ii) followed by crystallizing to thereby obtain roxithromycin with a desired level of impurity-H, e.g. of less than 0.5%.
(iii) dissolving the purified product thus obtained in step (ii) above in acetone and precipitating with water to thereby obtain the desired polymorph.

In accordance with a yet further aspect of the present invention there are provided novel crystallized forms of roxithromycin as several distinct solid state crystalline polymorphic forms differentiated by the positions of the atomic nuclei in the unit cell of the solidified compound to selectively provide roxithromycin with different macroscopic properties like thermal behavior, vapor permeability and solubility.

Importantly, it is found that the disclosed processes enable novel polymorphs to be obtained which meet the European pharmacopoeia monograph.

### DETAILED DESCRIPTION:

The process of purification in accordance with the present invention thus essentially involves the steps of at least partly dissolving the impure roxithromycin in selective solvent selected from ethanol, acetonitrile, aromatic solvents and mixtures of ethanol and acetonitrile and thereafter initiating crystallisation by cooling off or by addition e.g. of water. The dissolution may be in whole or in part.

In accordance with a preferred aspect, the method of the present invention comprises dissolving the impure roxithromycin at a temperature near to that of the reflux temperature of ethanol and then adding water slowly. Thereafter, the mixture is cooled slowly, in a controlled manner to approximately 0°C- 5°C. Preferably the cooling should be at a rate of 5°C per hour. Cooled mixture is then stirred for 30 minutes at 0°C- 5°C. (As an alternative to stirring, alternative agitation may be used, as the skilled person will understand in relation to each reference herein to stirring). The solution is filtered and the purified solid is washed with a mixture of ethanol and water, previously cooled to approximately 0°C- 5°C. The purified solid is dried at a temperature between 40° and 60°C.

The selective purification method herein described allows reducing in an efficient way the late eluting impurities present in the roxithromycin, especially the impurity H (i.e. roxithromycin B).

In accordance with another aspect the roxithromycin is dissolved in ethanol at a temperature of approximately 55-60°C. Thereafter, the mixture is cooled to room temperature slowly, e.g. approximately in one to two hours and then cooled to approximately 0°C- 5°C. (Room temperature is a temperature of from 20°C-35°C, e.g. of more than 25°C up to 35°C). The solution is filtered and the solid is washed with ethanol or ethanol and water previously cooled to approximately about 0°C- 5°C. The solid is dried at a temperature between 40° and 60°C.

In another aspect the purification method of the invention comprises dissolving the roxithromycin in a mixture of ethanol and acetonitrile, ranging from 5-95% and 95-5%, preferably 40-60%, at a temperature of approximately 55-60°C. Thereafter, the mixture is cooled slowly, in a controlled fashion until approximately 0°C- 5°C. Preferably the cooling should be at a rate of about 5°C. per hour. The mixture is then stirred for 30 minutes at about 0°C- 5°C. The solution is filtered and the purified solid washed with a mixture of ethanol and acetonitrile, previously cooled to approximately about 0°C- 5°C. The solid is dried at a temperature between 40° and 60°C.

In accordance with another aspect, the method of purification comprises dissolving/suspending the roxithromycin in toluene or xylene at a temperature of approximately 60-65°C. Thereafter, the mixture is cooled to room temperature( 20-35° C) slowly, e.g. approximately in one to two hours, and then cooled to 20-25°C. The solution is filtered and the solid is washed with toluene or xylene. The solid is dried at a temperature between 40° and 60°C.

Purification of roxithromycin is preferably effected in ethanol. In this case impure roxithromycin is suspended in ethanol, and stirred for about 1-4 hours at a temperature of between 40°C to 60°C. The suspension is cooled slowly to approximately 0°C- 5°C. with good stirring. Thereafter the mass is filtered and the purified solid is washed with ethanol previously cooled to approximately 0°C- 5°C. The solid is dried at a temperature between 40° and 60°C.

In accordance with yet another aspect a purification method comprises dissolving the impure roxithromycin in acetonitrile andheating this mixture upto 60-65°C for about 20-60 minutes. The mixture is slowly cooled to room temperature and stirred at this temperature. The solid is filtered and washed with acetonitrile previously cooled to approximately 0°C- 5°C. The solid is dried at a temperature between 40°C and 60°C.

In accordance with a yet further aspect the present invention provides two novel polymorphic forms of roxithromycin involving purification with the selective solvents in accordance with the invention. The novel polymorph Forms B and C are characterized as hereunder:

A crystalline polymorph of roxithromycin which exhibits a characteristic X-ray powder diffraction pattern with characteristic peak expressed in d-values (A°) at 10.54,8.95,8.49,8.03,7.55,7.31,6.61,6.22,6.06,5.85,5.50,5.40,5.25,5.10,4.97,4.69,4.55, 4.48,4.31,4.19,4.09,4.00,3.85,3.64,3.50,3.40,3.33,3.27,3.20,3.16,3.10,3.02,2.98,2.87,2 .76,2.70,2.66,2.61,2.56,2.50,2.42,2.36; herein designated as Form B.

The solid state ¹³C NMR spectra having chemical shift in parts per million (ppm) at 177.20, 174.21, 103.92, 100.58, 98.50, 87.20, 81.56, 78.00, 77.20, 76.30, 74.30, 71.25, 69.65, 68.90, 68.20, 62.02, 51.97, 48.43, 41.08, 35.84, 30.04, 28.90, 24.26, 21.70, 18.98, 17.68, 13.93, 12.24; herein designated as Form B.

A crystalline polymorph of roxithromycin which exhibits a characteristic X-ray powder diffraction pattern with characteristic peak expressed in d-values (A°) at 13.20,9.95,8.93,8.59,7.88,7.50,7.03,6.64,6.22,5.67,5.50,5.23,5.07,4.98,4.71,4.48,4.33, 4.22,4.07,4.00,3.84,3.63,3.38,2.87; herein designated as Form C.

The solid state ¹³C NMR spectra having chemical shift in parts per million (ppm) at 177, 176, 173.50, 105.50, 104.03, 101.06, 100.26, 85.70, 85.00, 83.00, 77.90, 77.25, 75.14, 74.52, 72.36, 70.40, 68.70, 62.03, 51.80, 46.70, 42.80, 40.70, 38.12, 36.12, 29.90, 28.30, 24.20, 21.34, 20.60, 18.51, 17.46, 13.18, 11.92; herein designated as Form C.

Furthermore, the present invention is directed to processes for the preparation of Forms B and C.

In accordance with another aspect the present invention is directed to highly pure crystalline Form A as characterized hereunder:

A crystalline polymorph of roxithromycin which exhibits a characteristic X-ray powder diffraction pattern with characteristic peak expressed in d-values (A°) at 10.5,8.94,8.46,8.01,7.54,7.31,6.64,6.19,6.06,5.85,5.50,5.39,5.25,5.09,4.98,4.69,4.55,4 .48,4.69,4.55,4.48,4.31,4.20,4.08,3.99,3.85,3.79,3.64,3.50,3.41,3.16,2.98,2.87,2.69,2. 36; herein designated as Form A

The solid state ¹³C NMR spectra having chemical shift in parts per million (ppm) at 177, 174, 106, 101, 99.12, 87.40, 82.20, 81.30, 80.50, 77.00, 76.00, 76.00, 75.80, 75.00, 73.4, 72.80, 71.37, 69.84, 68.80, 62.38, 53.08, 48.49, 43.33, 40.69, 38.13, 35.79, 31.60, 29.36, 24.25, 20.95, 20.15, 18.37, 13.87 and 13.20 herein designated as Form A.

A crystalline polymorph of roxithromycin which exhibits a characteristic X-ray powder diffraction pattern with characteristic peak expressed in d-values (A°) at 10.5,8.94,8.46,8.01,7.54,7.31,6.64,6.19,6.06,5.85,5.50,5.39,5.25,5.09,4.98,4.69,4.55,4 .48,4.69,4.55,4.48,4.31,4.20,4.08,3.99,3.85,3.79,3.64,3.50,3.41,3.16,2.98,2.87,2.69,2. 36; herein designated as Form A

The solid state ¹³C NMR spectra having chemical shift in parts per million (ppm) at 177, 174, 106, 101, 99.12, 87.40, 82.20, 81.30, 80.50, 77.00, 76.00, 76.00, 75.80, 75.00, 73.4, 72.80, 71.37, 69.84, 68.80, 62.38, 53.08, 48.49, 43.33, 40.69, 38.13, 35.79, 31.60, 29.36, 24.25, 20.95, 20.15, 18.37, 13.87 and 13.20 herein designated as Form A.

The above highly pure crystalline Form A is obtained using the very low impurity H Form B/C of the invention.

The roxithromycin described herein may be formulated into a pharmaceutical formulation, typically a composition additionally containing a pharmaceutically acceptable diluent, excipient or carrier. The formulation may be used to treat a bacterial or parasitic infection. It may be administered orally or parenterally. Further, such formulations may contain a combination of roxithromycin and another pharmaceutically active product.

The invention is illustrated in greater detail hereunder in relation to non-limiting exemplary illustrations.

### BRIEF DESCRIPTION OF THE FIGURES

1) Fig. 1 is characteristic X-ray power diffraction pattern for Form-A
2) Fig. 2 is characteristic X-ray power diffraction pattern for Form-B
3) Fig. 3 is characteristic X-ray power diffraction pattern for Form-C

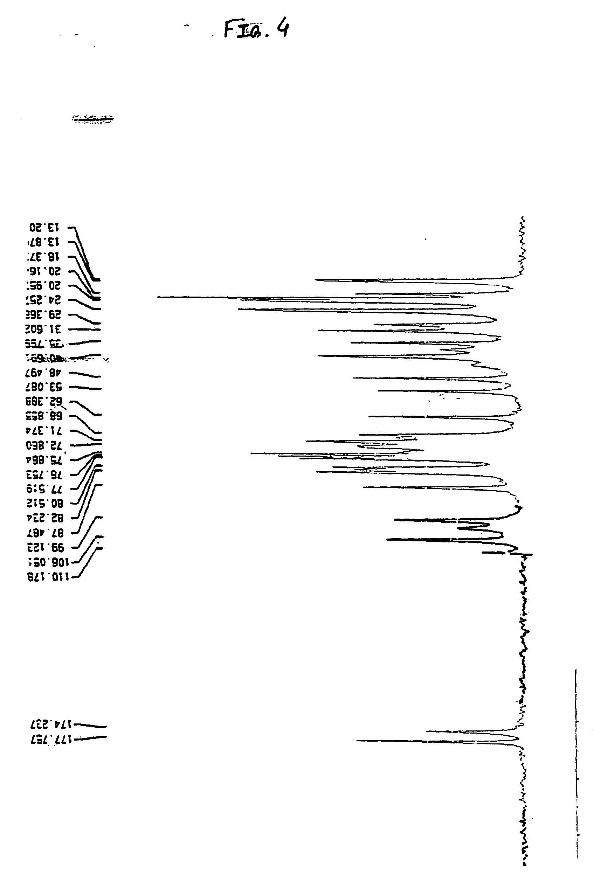

4) Fig. 4 is characteristic solid state NMR for Form-A
5) Fig. 5 is characteristic solid state NMR for Form-B
6) Fig. 6 is characteristic solid state NMR for Form-C

### PREPARATION OF CRUDE ROXITHROMYCIN:

### EXAMPLE A:

Erythromycin-9-oxime (50 gm) was mixed with acetone (250ml), the mixture was stirred for 10 minutes, cooled to 0-5°C. Sodium ethoxide (5.0 gm) was added followed by (methoxyethoxy) methyl chloride (8.6 ml), slowly with stirring, at 0-5°C. The reaction was monitored by TLC until erythromycin-9-ioxime disappears.
The reaction mixture was stirred at 0°C to 5°C for fifteen minutes and slowly raised up to ambient. Water (100 ml) was added to the reaction mixture and the reaction mixture became clear. Water (200ml) was added till turbidity appeared, when water addition was stopped. The reaction mixture was stirred for half an hour and other water (200ml) was added to the reaction mixture. After two hours the solid was filtered and washed with acetone: water (50:50) mixture, dried at 50-60°C, to give crude roxithromycin (43.5gm).

The crude roxithromycin is obtained by following the reference Example A and the purification process is explained in Example 1 to Example 5 to obtain pure roxithromycin.

In reference Example A one can use a base such as potassium t-butoxide or another alkali metal alcoholate (especially alkoxide) in place of sodium ethoxide.

### EXAMPLE 1

### Preparation of polymorphic Form C

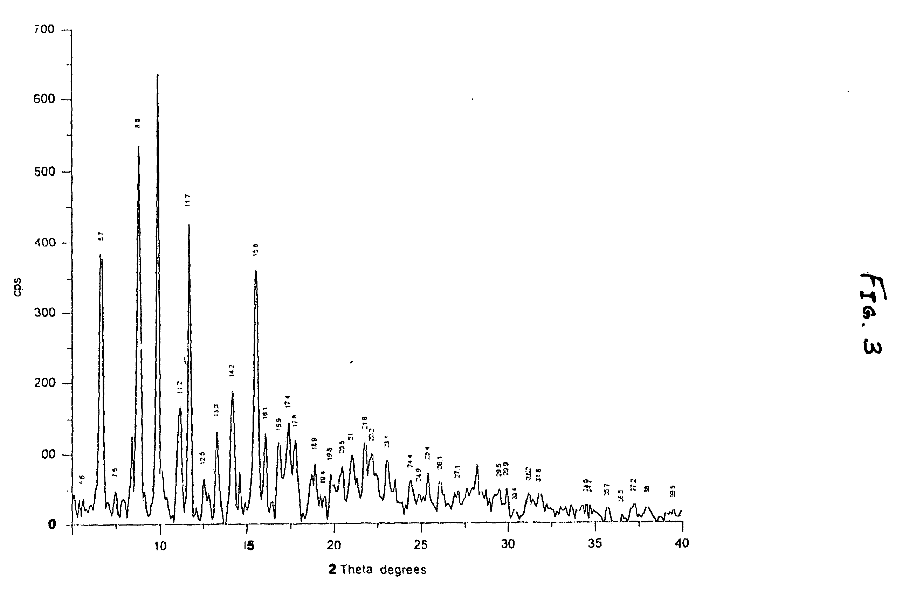

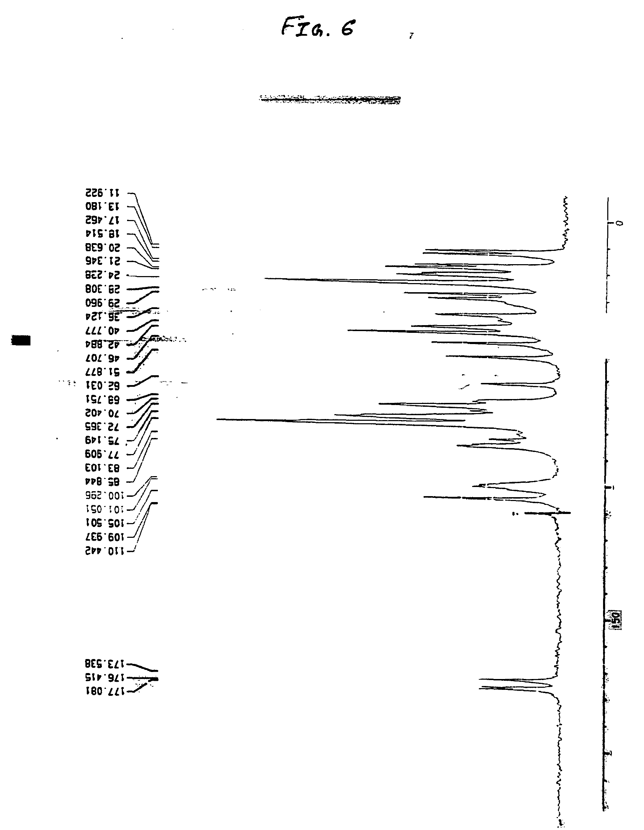

100 ml. Ethanol are added to 100g of roxithromycin with 96.2% purity by h.p.l.c. and 0.6-1 % of the impurity H, heating until dissolution. The solution is slowly cooled until the beginning of turbidity and seeded with good quality roxithromycin. Thereafter it is stirred for half an hour maintaining the temperature and then cooling with control until 0°C. The solid is filtered and washed with a mixture of ethanol previously cooled to 0°C. After drying, 84g of a product with 98.12% purity and 0.36% of the impurity H is obtained. This leads to the preparation of Form C. X-ray powder diffraction and Solid State NMR studies shows the product to be polymorphic From C (Fig 3, 6)

### EXAMPLE 2

### Preparation of polymorphic Form B

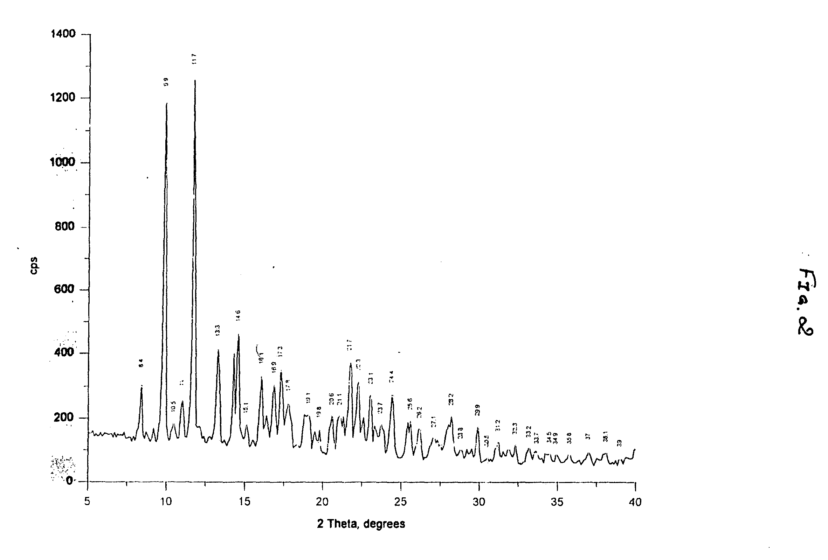

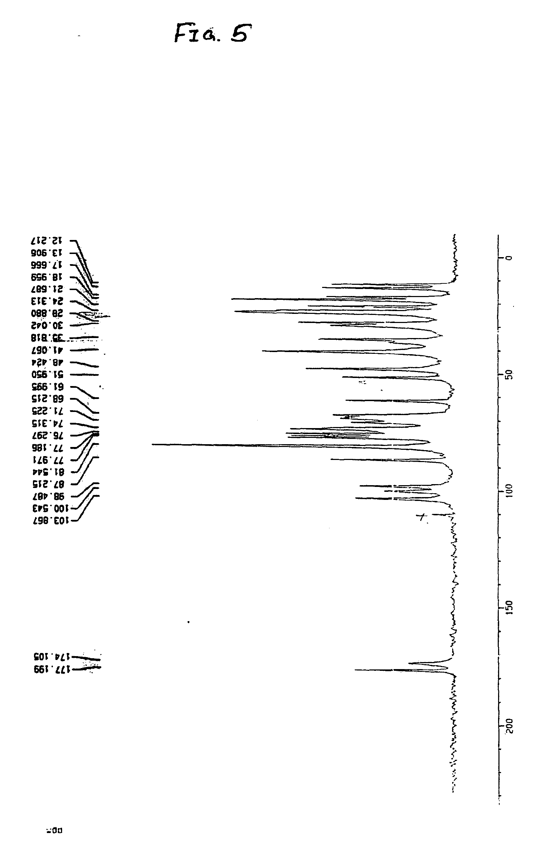

100g roxithromycin with 96.5% purity by h.p.l.c. and 0.6-1.0 % of the impurity H, are suspended in 100ml ethanol. The mixture is heated up to 60°C and maintained the temperature and slowly add 30ml pure water. First the mixture is cooled to room temperature, and then cooled to 0°C (cooling rate 5°C/hours). Stir the mixture for thirty minutes. The solid is filtered and washed with a mixture of ethanol and water, previously cooled to 0°C. 82 g of a product with 98.13% purity and 0.08% of the impurity H. This leads to the preparation of Form B. X-ray powder diffraction and Solid State NMR studies shows the product to be polymorphic From B ( Fig 2, 5).

### EXAMPLE 3

100g of roxithromycin with 96.2% purity by h.p.l.c. and 0.6-1.0 % of the impurity H are suspended in 200ml Acetonitirle and stirred at 60-65°C for thirty minutes. The mixture is cooled to room temperature over a period of 6-7 hrs, then cooled to 0°C and stirred for 30 minutes. The solid is filtered and washed with acetonitrile previously cooled to 0°C. After drying, 88g of a product with 98.3% purity and 0.3% of the impurity H is obtained.

### EXAMPLE 4

### Preparation of polymorphic Form B

100 ml. Toluene is added to 100g of roxithromycin (dry) with 96.2% purity by h.p.l.c. and 0.6-1 % of the impurity H and then heated until dissolution. The solution is cooled until the beginning of turbidity and is then seeded with good quality roxithromycin. Thereafter it is stirred for half an hour maintaining the temperature and then cooled with control until room temperature. The solid is filtered and washed with the same solvent; 70g of a product with 98.5% purity and 0.4% of impurity H is obtained. This leads to the preparation of Form B. X-ray powder diffraction and Solid State NMR studies shows the product to be polymorphic Form B ( Fig 2, 5)

### EXAMPLE 5

### Preparation of polymorphic Form C

100g of roxithromycin with 96.2% purity by h.p.l.c. and 0.6-1.0 % of the impurity H is suspended in 200ml acetonitrile : ethanol (Ratio 1:1) and stirred at 60-65°C for thirty minutes. The mixture is cooled to room temperature over a period of 6-7 hrs. Then it is cooled to O°C and stirred for the 30 minutes. The solid is filtered and washed with acetonitrile previously cooled to O°C. After drying, 88g of a product with 98.3% purity and 0.3% of the impurity H. This leads to the preparation of Form C. X-ray powder diffraction and Solid State NMR studies shows the product to be polymorphic From C (Fig 3, 6)

### EXAMPLE 6

### Preparation of polymorphic Form A

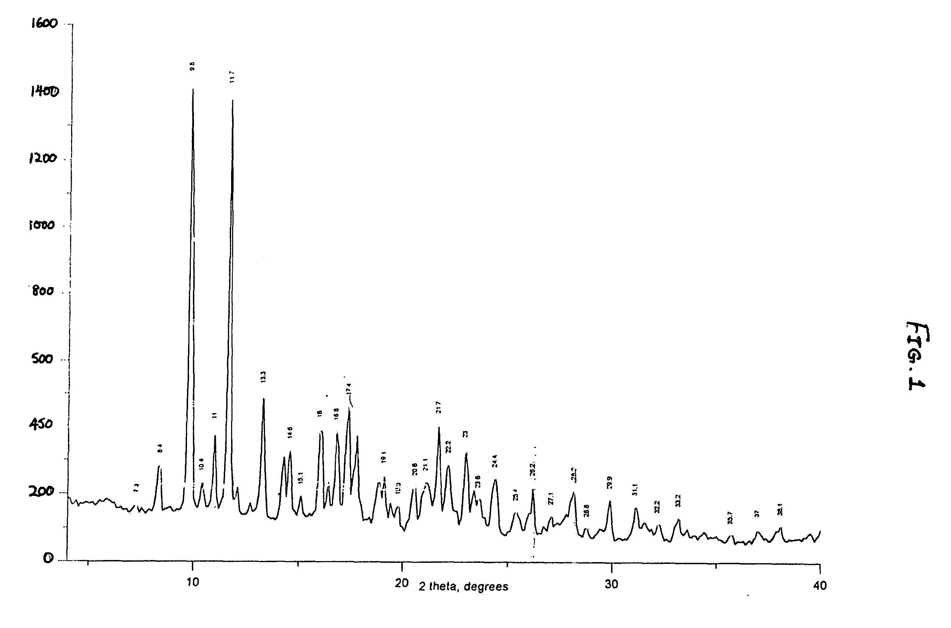

70 g of roxithromycin obtained by Examples 1-5 having a purity of 97.50- 98.20 % and 0.1 to 0.4 % of Impurity H is dissolved in 210 ml acetone, filtered to provide a clear solution and reprecipitated by adding 280 ml pure water. The solid is washed with 1:1 acetone: water. 66 g of pure roxithromycin obtained having a purity of 98.8-99.5% purity of roxithromycin. This leads to the preparation of Form A. X-ray powder diffraction and Solid State NMR studies shows the product to be polymorphic Form A (Fig 1, 3)

For finding out the efficacy of the selective solvents such as ethanol, aqueous ethanol, acetonitrile and toluene of the process of the invention vis-à-vis solvents used in the prior art such as methanol and acetone for removing the impurity -H in roxithromycin, experiments are carried out using above mentioned solvents .For the purpose the above solvents were used and purification of crude roxithromycin was carried out following the process as detailed in Example 1 to 5 and the results are summarized in Table-1A and 1B hereunder:

**Table -1A**

| Ex.No. | | Purity of Roxithromycin | Impurity H |
|---|---|---|---|
| A1. | Crude | 96.36 | 0.616 |
| A2. | Methanol ( Prior art) | 98.26 | 0.604 |
| A3. | Acetonitrile *(Less than 0.1 % Moisture)* | 98.33 | 0.41 |
| A4. | Ethanol *) (Less than 2.0 % Moisture)* | 98.26 | 0.31 |
| A5. | Ethanol-water *( The ratios of Ethanol : Water is 1: 0.3 v*/*v)* | 98.13 | 0.08 |
| A6. | Acetonitrile-ethanol*)( The ratios of Acetonitrile -Ethanol is 1: 1 v*/*v)* | 98.32 | 0.05 |
| A7. | Toluene *(Less than 0.1 % Moisture)* | 98.77 | 0.342 |

**TABLE 1B**

| Ex. No. | | Purity of Roxithromycin | Impurity H |
|---|---|---|---|
| B1. | Crude | 96.20 | 1.157 |
| B2. | Methanol | 98.32 | 0.997 |
| B3. | Acetone | 97.75 | 0.849 |
| B4. | Ethanol | 98.12 | 0.366 |
| B5. | Acetonitrile ethanol *( The ratios of Acetonitrile - Ethanol is 1: 1 v*/*v)* | 98.323 | nil |

The above Tables-1A and 1B clearly reveal that the conventional solvents for roxithromycin purification i.e. methanol and acetone fail to purify Impurity-H to the desired level but the selective solvents ethanol, ethanol-water, actetonitrile, actetonitrile-ethanol and toluene are capable of recovering Impurity-H to the desired extend of less than 0.5% as prescribed by the European Pharmacopoeia limits. The described process thus improves the preparation of Roxithromycin in terms of effective removal of Impurity-H.

The novel polymorph Form B can be obtained by crystallizing roxithromycin from an aromatic solvent such as toluene. Roxithromycin is combined with toluene and dissolved by heating the solvent before the solvent is cooled slowly *(what is slowly?)*. The polymorph Form B can also be obtained by dissolving the roxithromycin in ethanol and crystallizing it by adding water to the reaction mixture.

Form B has following X-ray powder diffraction pattern with characteristic peak expressed in d-values (A°) at 10.54, 8.95, 8.49, 8.03, 7.55, 7.31, 6.61, 6.22, 6.06, 5.85, 5.50, 5.40, 5.25, 5.10, 4.97, 4.69, 4.55, 4.48, 4.31, 4.19, 4.09, 4.00, 3.85, 3.64, 3.50, 3.40, 3.33, 3.27, 3.20, 3.16, 3.10, 3.02, 2.98, 2.87, 2.76, 2.70, 2.66, 2.61, 2.56, 2.50, 2.42, 2.36

The novel polymorph Form B has solid state ¹³C NMR spectra chemical shift in parts per million (ppm) at 177.20, 174.21, 103.92, 100.58, 98.50, 87.20, 81.56, 78.00, 77.20, 76.30, 74.30, 71.25, 69.65, 68.90, 68.20, 62.02, 51.97, 48.43, 41.08, 35.84, 30.04, 28.90, 24.26, 21.70, 18.98, 17.68, 13.93, 12.24.

The novel polymorph Form B is obtained by crystallizing roxithromycin from ethanol: water mixture or from aromatic solvent such as toluene. The graphs are shown in Fig. 2 and 5 respectively for XRD and ¹³ C Solid State NMR.

The Form C can be obtained in accordance with an embodiment by dissolving Roxithromycin in ethanol or ethanol: acetonitrile mixtures under heating and cooling slowly at room temperature (20-35°C) to obtain novel polymorph Form C.

Form C has the following X-ray powder diffraction pattern with characteristic peak expressed in d-values (A°) at 13.20, 9.95, 8.93, 8.59, 7.88, 7.50, 7.03, 6.64, 6.22, 5.67, 5.50, 5.23, 5.07, 4.98, 4.71, 4.48, 4.33, 4.22, 4.07, 4.00, 3.84, 3.63, 3.38, 2.87.

The solid state ¹³C NMR spectra of Form C has chemical shift in parts per million (ppm) at 177, 176, 173.50, 105.50, 104.03, 101.06, 100.26, 85.70, 85.00, 83.00, 77.90, 77.25, 75.14, 74.52, 72.36, 70.40, 68.70, 62.03, 51.80, 46.70, 42.80, 40.70, 38.12, 36.12, 29.90, 28.30, 24.20, 21.34, 20.60, 18.51, 17.46, 13.18, 11.92.

The graphs are as shown in Fig. 3 and 6 respectively for XRD and ¹³ C Solid State NMR.

As will be apparent from the above the selective purification of Impurity-H to less than 0.5% achieved involving the selective solvents of the invention provide for novel polymorphs with distinguished XRD values and ¹³ C solid state NMR.

The highly pure polymorph Form A is obtained by crystallizing any of the above Forms B or C of the invention using acetone: water mixture. For the purpose, the roxithromycin Form B/C of the invention is dissolved in acetone and then the addition of water causes crystallization.

Form A has following X-ray powder diffraction pattern with characteristic peak expressed in d-values (A°) at 10.5, 8.94, 8.46, 8.01, 7.54, 7.31, 6.64, 6.19, 6.06, 5.85, 5.50, 5.39, 5.25, 5.09, 4.98, 4.69, 4.55, 4.48, 4.69, 4.55, 4.48, 4.31, 4.20, 4.08, 3.99, 3.85, 3.79, 3.64, 3.50, 3.41, 3.16, 2.98, 2.87, 2.69, 2.36.

The solid state ¹³C NMR spectra of Form A has a chemical shift in parts per million (ppm) at 177, 174, 106, 101, 99.12, 87.40, 82.20, 81.30, 80.50, 77.00, 76.00, 76.00, 75.80, 75.00, 73.4, 72.80, 71.37, 69.84, 68.80, 62.38, 53.08, 48.49, 43.33, 40.69, 38.13, 35.79, 31.60, 29.36, 24.25, 20.95, 20.15, 18.37, 13.87 and 13.20.

## Claims

1. A process for the purification of roxithromycin with high level of impurity -H comprising:
(i) subjecting the impure roxithromycin to at least partial dissolution and/or suspension in solvent selected from ethanol, acetonitrile, aromatic solvents and mixtures of ethanol and acetonitrile;
(ii) followed by crystallizing to thereby obtain roxithromycin with desired level of impurity-H of less than 0.5%.

2. A process for the purification of roxithromycin with high level of impurity -H comprising
(i) subjecting the impure roxithromycin to at least partial dissolution and/or suspension in solvent selected from ethanol, acetonitrile, aromatic solvents and mixtures of ethanol and acetonitrile;
(ii) followed by crystallizing to thereby obtain roxithromycin with desired level of impurity-H of less than 0.5%.
(iii) dissolving the purified product thus obtained in step (ii) above in acetone and precipitating with water.

3. A process according to anyone of claims 1 or 2, wherein the impure roxithromycin is at least partially dissolved and suspended in ethanol with heating and the purified product obtained by cooling the mixture.

4. A process according to anyone of claims 1 or 2, wherein the impure roxithromycin is at least partially dissolved and suspended in ethanol and the purified product is obtained by adding water.

5. A process according to anyone of claims 1 or 2, wherein the impure roxithromycin is at least partially dissolved and suspended in acetonitrile with heating and the purified product obtained by cooling the mixture.

6. A process according to anyone of claims 1 or 2, wherein the impure roxithromycin is at least partially dissolved and suspended in the mixture of ethanol and acetonitirle with heating and the purified product obtained by cooling the mixture.

7. A process as claimed in claim 6, wherein the amount of ethanol and acetonitrile in the mixture range from 5-95%, preferable 40-60%.

8. A process according to anyone of claims 1 or 2, wherein the impure roxithromycin is at least partially dissolved and suspended in an aromatic solvent, with heating and the purified product is obtained by cooling the mixture.

9. A process according to claim 8, wherein the aromatic solvent used is selected from the group of toluene and xylene.

10. A process according to anyone of claims 1 or 2 wherein the crystallisation is initiated by cooling off and/or by addition of water.

11. A process according to anyone of claims 1 or 2 comprising dissolving the impure roxithromycin in ethanol at a temperature near to that of the reflux temperature of ethanol and then adding water slowly), cooling the mixture slowly, in a controlled manner upto approximately about 0°C- 5°C., filtering the solution to recover precipitate, washing the precipitate with a mixture of ethanol and water, previously cooled to approximately 0°C- 5°C and drying the purified solid at a temperature between 40° and 60°C.

12. A process according to claim 11 wherein the cooling is done at a rate of of 5°C. per hour.

13. A process according to anyone of claims 1 or 2 comprising dissolving the impure roxithromycin in ethanol at a temperature of approximately 55-60°C, cooling the mixture to room temperature approximately in one to two hours, cooling to approximately 0°C- 5°C, filtering the solution to recover precipitate, washing the solid with ethanol or ethanol-water previously cooled to approximately about 0°C- 5°C and drying the solid at a temperature between 40° and 60°C.

14. A process according to anyone of claims 1 or 2 comprising dissolving the roxithromycin in a mixture of ethanol and acetonitrile, ranging from 5-95% and 95-5%, preferably 40-60%, at a temperature of approximately 55-60°C, cooling the mixture to approximately 0°C- 5°C., filtering the solution to recover precipitate, washing the precipitate with a mixture of ethanol and acetonitrile, previously cooled to approximately about 0°C- 5°Cand drying the solid at a temperature between 40° and 60°C.

15. A process according to anyone of claims 1 or 2 comprising dissolving/suspending the roxithromycin in toluene or xylene at a temperature of approximately 60-65°C, cooling the mixture to room temperature approximately in one to two hours, cooling the mixture to 20-25°C, filtering the solution to recover precipitate, washing the precipitate with toluene or xylene, and drying the solid at a temperature between 40° and 60°C.

16. A process according to anyone of claims 1 or 2 comprising suspending impure roxithromycin in ethanol, stirring for about 1-4 hours at a temperature of between 40°C to 60°C, cooling the suspension to approximately 0°C- 5°C. with stirring, filtering the solution to recover precipitate, washing the precipitate with ethanol previously cooled to approximately 0°C- 5°C, and drying the solid at a temperature between 40° and 60°C.

17. A process according to anyone of claims 1 or 2 comprising dissolving the impure roxithromycin in acetonitrile, heating this mixture up to 60-65°C for about 20-60 minutes, cooling the mixture to room temperature and stirring it at this temperature, filtering the solution to recover precipitate, washing the precipitate with acetonitrile previously cooled to approximately 0°C- 5°Cand drying the solid at a temperature between 40°C and 60°C.

18. A process according to any preceding claim which further comprises formulating the roxithromycin into a pharmaceutical formulation.

19. Crystalline polymorphic forms of roxithromycin selected from Form B and Form C obtained by the process according to claim 1.

20. Crystalline Form B roxithromycin as claimed in claim 19 which exhibits a characteristic X-ray powder diffraction pattern with characteristic peaks expressed in d-values (A°) at 10.54, 8.95, 8.49, 8.03, 7.55, 7.31, 6.61, 6.22, 6.06, 5.85, 5.50, 5.40, 5.25, 5.10, 4.97, 4.69, 4.55, 4.48, 4.31, 4.19, 4.09, 4.00, 3.85, 3.64, 3.50, 3.40, 3.33, 3.27, 3.20, 3.16, 3.10, 3.02, 2.98, 2.87, 2.76, 2.70, 2.66, 2.61, 2.56, 2.50, 2.42, 2.36.

21. Crystalline Form B roxithromycin as claimed in claim 20 further **characterized by** ¹³ C solid state NMR spectra having chemical shifts in parts per million (ppm) : 177.20, 174.21, 103.92, 100.58, 98.50, 87.20, 81.56, 78.00, 77.20, 76.30, 74.30, 71.25, 69.65, 68.90, 68.20, 62.02, 51.97, 48.43, 41.08, 35.84, 30.04, 28.90, 24.26, 21.70, 18.98, 17.68, 13.93, 12.24

22. Crystalline Form C roxithromycin which exhibits a characteristic X-ray powder diffraction pattern with characteristic peaks expressed in d-values (A°) at 13.20, 9.95, 8.93, 8.59, 7.88, 7.50, 7.03, 6.64, 6.22, 5.67, 5.50, 5.23, 5.07, 4.98, 4.71, 4.48, 4.33, 4.22, 4.07, 4.00, 3.84, 3.63, 3.38, 2.87.

23. Crystalline Form C roxithromycin as claimed in claim 22 **characterized by** ¹³ C solid state NMR spectra having chemical shifts in parts per million (ppm): 177, 176, 173.50, 105.50, 104.03, 101.06, 100.26, 85.70, 85.00, 83.00, 77.90, 77.25, 75.14, 74.52, 72.36, 70.40, 68.70, 62.03, 51.80, 46.70, 42.80, 40.70, 38.12, 36.12, 29.90, 28.30, 24.20, 21.34, 20.60, 18.51, 17.46, 13.18, 11.92

24. A highly purified crystalline Form A of roxythromycin obtained by the process of claim 2 which exhibits selective Form A, X-ray powder diffraction pattern with characteristic peak expressed in d-values (A°) at 10.5, 8.94, 8.46, 8.01, 7.54, 7.31, 6.64, 6.19, 6.06, 5.85, 5.50, 5.39, 5.25, 5.09, 4.98, 4.69, 4.55, 4.48, 4.69, 4.55, 4.48, 4.31, 4.20, 4.08, 3.99, 3.85, 3.79, 3.64, 3.50, 3.41, 3.16, 2.98, 2.87, 2.69, 2.36; and the solid state ¹³C NMR spectra of Form A having chemical shift in parts per million (ppm) at 177, 174, 106, 101, 99.12, 87.40, 82.20, 81.30, 80.50, 77.00, 76.00, 76.00, 75.80, 75.00, 73.4, 72.80, 71.37, 69.84, 68.80, 62.38, 53.08, 48.49, 43.33, 40.69, 38.13, 35.79, 31.60, 29.36, 24.25, 20.95, 20.15, 18.37, 13.87 and 13.20.

25. A pharmaceutical formulation comprising the roxithromycin of any of claims 19 to 24, and optionally further containing another pharmaceutically active product.
